# EUROPEAN PATENT SPECIFICATION

(11) **EP 2 636 439 B1**
(45) Date of publication and mention of the grant of the patent: **11.05.2016**
(21) Application number: 11824296.5
(22) Date of filing: 13.04.2011
(51) Int. Cl.: C10K 1/04, C10K 1/08, B01D 47/06, C10K 1/02, C07C 1/20, C10G 3/00, B01D 21/26

(54) **METHOD FOR CLEANING AND SEPARATING MTO REACTION GAS CONTAINING CATALYST MICROPOWDERS**
VERFAHREN ZUR REINIGUNG UND TRENNUNG VON MTO-REAKTIONSGAS MIT KATALYSATOR-MIKROPULVERN
PROCÉDÉ POUR LE NETTOYAGE ET LA SÉPARATION D'UN GAZ DE RÉACTION MTO CONTENANT DES MICROPOUDRES CATALYTIQUES

(30) Priority: 05.11.2010 CN 201010533902
(43) Date of publication of application: 11.09.2013
(73) Proprietor: East China University Of Science And Technology, Shanghai 200237 (CN)
(72) Inventor: YANG, Qiang, Shanghai 200237 (CN); WANG, Hualin, Shanghai 200237 (CN); LI, Zhiming, Shanghai 200237 (CN); ZHANG, Yanhong, Shanghai 200237 (CN); CUI, Xin, Shanghai 200237 (CN); XU, Dejian, Shanghai 200237 (CN); LV, Wenjie, Shanghai 200237 (CN); XU, Xiaomei, Shanghai 200237 (CN)
(74) Representative: V.O.
(86) International application number: PCT/CN2011/072708
(87) International publication number: WO 2012/058901

(56) References cited:
- EP-A2- 2 163 310
- WO-A1-2008/076620
- WO-A1-2008/082951
- AU-B2- 689 534
- CA-A1- 1 107 941
- CN-A- 101 352 620
- CN-A- 101 352 621
- CN-A- 101 384 685
- US-A1- 2002 004 534
- US-A1- 2005 234 281
- ANONYMOUS: 'WET SCRUBBERS' MIKROPUL, [Online] 01 March 2009, page 8PP, XP055160891 Retrieved from the Internet: <URL:http://www.mikropul.com/uploads/pdf/we t_scrubbers.pdf> [retrieved on 2015-01-09]

## Description

### Technical Field

The invention pertains to the technical field of coal chemical engineering, and in particular, relates to a process for optimized combination of purification and separation of MTO reaction gas containing catalyst fine powder according to claim 1.

### Background Art

MTO (Methanol To Olefins) refers to a technology for manufacturing lower olefin (ethylene, propylene, butene, etc.) from methanol, the main principle of which is production of methanol from synthetic gas originating from coal, and subsequent conversion of methanol to lower olefin (with ethylene and propylene as the major components). Lower olefin is the most important fundamental raw material for organic chemical engineering, with vigorous market demand. The traditional production approach of lower olefin (by cracking refined oil to produce light hydrocarbons and naphtha) is to be challenged by the tight supply of raw material due to depleting oil resource and stubbornly high oil price. Successful industrialization of MTO technology would open a totally new technical route for modern coal chemical engineering. Owing to resource and economic advantages, this technology is quite significant for energy development. The reaction part of MTO comprises a reactor and a regenerator, and the product purifying part comprises a quench tower, a water scrubbing tower, an alkali scrubbing tower and a drying tower, with the remaining being the product separation part. The reactor commonly used is a fluid bed reactor in which methanol as the reactant and the olefin products are present in gas phase, and the reaction gas entraining catalyst goes to the product purifying part after passing through three or four levels of cyclone separators at the top of the reactor. Due to the limitation of the cyclone separators, the reaction gas still entrains a small amount of catalyst fine powder with particle size of less than 10µm or 5µm even after it has been separated by cyclone separation. The catalyst fine powder has to be removed effectively at the product purifying part. Moreover, MTO catalyst is relatively expensive. Thus, it is desirable to recover the catalyst by an effective means.

US 6166282 discloses a MTO fluid bed reactor wherein a set of cyclone separators are installed on the top of the reactor to separate solid catalyst particles from the product effluent and thus prevent the catalyst particles from being carried out of the reactor. Albeit the reaction gas stream passes through several cyclone separating devices, there are still catalyst particles that leave the reactor together with the product effluent. In a quench tower and a water scrubbing tower, this catalyst fine powder is separated from the reaction gas product along with the quench water and the scrubbing water respectively, and enters the water circulating system with the quench water and the scrubbing water, leading to abrasion and clogging of downstream devices, as well as shortened operating cycle of the apparatus.

US 5744680A discloses a process for preparing light hydrocarbons from oxides wherein the stream containing catalyst is discharged in a wet scrubbing step. US 6870072 also discloses removal of catalyst from a product effluent using a wet scrubbing zone. This patent application uses a method wherein a concentrated liquid containing catalyst is discharged from the apparatus. This not only results in waste of scrubbing liquid (quench water/scrubbing water), but also fails in effective recovery of catalyst fine powder.

For MTO quench water, US 0234281 and CN 1942558A disclose an apparatus combining one or more solid-liquid cyclone separators or hydrocyclone separators working in series or in parallel, to separate and recycle catalyst. Since the product stream from the reactor has already passed through several gas-solid cyclone separating devices in the reactor, the particle size of the catalyst particles in the MTO quench water is quite small (typically no more than 5µm), and the particles in the scrubbing water are even smaller (smaller than 3µm). However, the size of the particles that can be captured effectively by a conventional solid-liquid cyclone or hydrocyclone separator is about 5-10µm. This means that the apparatus can only remove the catalyst which runs off under abnormal operating conditions wherein the cyclone separators on the top of the reactor fail or work unsteadily, etc. In other words, solid particles smaller than 5µm are difficult to remove under normal operating conditions of the cyclone separators when the facility runs steadily. Therefore, the solid-liquid cyclone or hydrocyclone separators in the above mentioned patent applications can only be used in an accident of catalyst run-off, and the predicted effect does not occur in normal operation.

EP2163310 discloses a method for for purifying MTO (methanol-to-olefin process) quench water and scrubbing water via mini-hydrocyclone separation. However the scrubbing effect of the quench tower does not sufficiently separate the catalyust particles from the reaction gas such that minor catalyst partociles will be entrained by the reaction gas to the subsequent water scrubbing tower. This results in pollution of the scrubbing water in the water scrubbing tower and the requirement of an additional device to treat the scrubbing water. This increases the maintenance cost and energy consumption of the system.

All of the foregoing patent applications relate to a process or apparatus for controlling the catalyst fine powder entrained in the reaction gas or purifying the scrubbing liquid (quench water/scrubbing water) after the reaction gas entraining catalyst fine powder is scrubbed. An economic and effective process featuring simple operation and long operating cycle for purifying and cooling reaction gas entraining catalyst fine powder, separating, clarifying and recycling scrubbing liquid (quench water/scrubbing water), and recovering and reusing catalyst fine powder in solid form is not available yet in the art.

Therefore, it is an urgent need in the art to develop an economic and effective process featuring simple operation and long operating cycle for purifying and cooling reaction gas entraining catalyst fine powder, separating, clarifying and recycling scrubbing liquid (quench water/scrubbing water), and recovering and reusing catalyst fine powder in solid form.

### Summary

The invention provides a novel process for optimized combination of purification and separation of MTO reaction gas containing catalyst fine powder, so as to overcome the drawbacks in prior art.

In one aspect, the invention provides a process for optimized combination of purification and separation of MTO reaction gas containing catalyst fine powder, comprising
(a) scrubbing, purifying and cooling the MTO reaction gas containing catalyst fine powder by spray scrubbing with atomized liquid drops or dynawave scrubbing, and separating and recovering the mist entrained in the scrubbed reaction gas before it leaves the scrubbing tower (quench tower/water scrubbing tower) and the catalyst fine powder that is not scrubbed off by using a cyclone tube or a cyclone plate, so as to purify the reaction gas containing catalyst fine powder;
(b) subjecting the scrubbing liquid (quench water/scrubbing water) containing catalyst after the scrubbing to solid-liquid separation, in a sealed enclosure by micro-cyclone separation or filtration separation, so as to remove catalyst particles in the scrubbing liquid (quench water/scrubbing water), wherein the solid content of the scrubbing liquid before separation is 30-400 mg/L, and that after separation is 10-50 mg/L and wherein the scrubbing liquid containing catalyst fine powder after separation is 1-10 vol.% of the flow of the scrubbing liquid containing catalyst fine powder;
(c) treating the purified scrubbing liquid (quench water/scrubbing water) after the solid-liquid separation by removing methanol by subsequent heat exchange, stripping and cooling in subsequent devices and then recycling the liquid, so as to ensure the continuous and steady operation of subsequent heat exchangers and strippers;
(d) further concentrating the concentrated liquid containing catalyst after solid-liquid separation, so as to obtain higher concentration of before the catalyst slurry;
(e) treating the further concentrated catalyst slurry by drying or centrifugal dewatering, so as to recover the catalyst fine powder in solid form;
(f) sending the reaction gas generated by drying to a torch for burning or recovering it by condensation in a condenser; and
(g) sending the purified scrubbing liquid (quench water/scrubbing water) generated in the process of centrifugal dewatering to the scrubbing tower (quench tower/water scrubbing tower) for repeated use.

In a preferred embodiment, the catalyst fine powder entrained in the MTO reaction gas containing catalyst fine powder has a content of 1-500mg/m³ and a particle size of 0.1-30µm, and the catalyst skeletal density is 1.1∼3 kg/m³.

The scrubbing, purification and cooling of the MTO reaction gas containing catalyst fine powder is carried out by spray scrubbing with atomized liquid drops or dynawave scrubbing; and, the separation and recovery of the mist entrained in the scrubbed reaction gas and the catalyst fine powder that is not scrubbed off is carried out using a cyclone tube or a cyclone plate.

The solid-liquid separation of the scrubbing liquid containing catalyst fine powder is carried out by micro-cyclone separation or filtration, wherein the solid content of the scrubbing liquid before separation is 30-400mg/L, and that after separation is 10-50mg/L.

The purified scrubbing liquid after separation is reused after removing methanol by subsequent heat exchange, stripping and cooling, and the scrubbing liquid containing catalyst fine powder after separation is further concentrated, wherein the scrubbing liquid containing catalyst fine powder after separation is 1-10 vol.% of the flow of the scrubbing liquid containing catalyst fine powder.

In another preferred embodiment, the scrubbing liquid containing catalyst fine powder after separation is further concentrated to a solid content of 30-70 vol.% by coupled separation of micro-cyclone separation with gravity settling or micro-cyclone separation with filtration and gravity settling.

In another preferred embodiment, the concentrated scrubbing liquid containing catalyst fine powder after separation with a solid content of 30-70 vol.% is dried or dewatered in a centrifuge, wherein the drying is spray granulation drying or steam rotary tube drying, the dried catalyst is recovered, and the dried gas is sent to a torch or recovered after condensed.

In another preferred embodiment, after the concentrated scrubbing liquid containing catalyst fine powder after separation with a solid content of 30-70 vol.% is dried, the water content of the catalyst particles is no more than 5 vol.%; alternatively, after the concentrated scrubbing liquid containing catalyst fine powder after separation with a solid content of 30-70 vol.% is dewatered in a centrifuge, the water content of the catalyst particles is no more than 10 vol.%.

In another preferred embodiment, using this method, more than 99% of the catalyst particles in the MTO reaction gas containing catalyst fine powder is removed, more than 98% of the purified scrubbing liquid after separation is recycled, more than 96% of the catalyst fine powder in the scrubbing liquid containing catalyst fine powder after separation is recovered, and the energy consumption for operating the system is no more than 0.6MPa.

### Brief Description of Drawings

Fig. 1 is a schematic flow chart of combined purification and separation of MTO reaction gas containing catalyst fine powder according to an embodiment of the invention.
Fig. 2 is a schematic flow chart of combined purification and separation of MTO reaction gas containing catalyst fine powder according to another embodiment of the invention.
Fig. 3 is a schematic flow chart of combined purification and separation of MTO reaction gas containing catalyst fine powder according to still another embodiment of the invention.
Fig. 4 is a schematic flow chart of combined purification and separation of MTO reaction gas containing catalyst fine powder according to yet another embodiment of the invention.

### Specific Modes for Carrying Out the Embodiments

After extensive and intensive study, the inventors have made the following findings. For the catalyst fine powder entrained in MTO reaction gas during purifying operation, which is characterized by small particle size (1-10µm under normal operation state and 1-50µm under abnormal state), small amount of solid particles and high demand for purification (no less than 98% of the catalyst particles are required to be removed), a scrubbing-cycloning coupled separation method is first used to separate the catalyst fine powder entrained in the reaction gas. Since the catalyst fine powder entrained in the reaction gas is the catalyst fine powder that can not be separated by the third stage cyclone in a catalytic cracking unit, and the reaction gas has high flow and high temperature, neither an electrostatic method nor a precision filtration method can be used to remove the dust (catalyst particles). Therefore, the catalyst fine powder has to be removed by a wet scrubbing method. Study on the phenomenon that the catalyst particles which have not been scrubbed off will be encapsulated by liquid membrane during scrubbing has enlightened the inventors on the use of a gas-liquid cyclone separation method to separate the mist and catalyst particles entrained in the reaction gas before it leaves the scrubbing tower (quench tower/water scrubbing tower), as further separation of the reaction gas. Secondly, as to the purification process of the scrubbing liquid (quench water/scrubbing water), study has been made on the characteristics of the scrubbing liquid (quench water/scrubbing water), a solid-liquid system which has high operating flux and small solid particle size (1-10µm, most smaller than 5µm), and it has been found that the most cost-effective method is the use of a cyclone separator. However, due to its cut diameter which is larger than 5µm, it is very difficult for a conventional cyclone or hydrocyclone separator to remove solid particles smaller than 5µm under normal operating conditions. On the other hand, a high precision backwash filter with a separation precision of 1-2µm has the problems of high manufacturing cost, large occupation area, easy clogging and the like, leading to undesirable effect of use under such operating conditions. Fortunately, a micro-cyclone separator has the advantages of wide applicability, simple structure, strong adaptability, easy maintenance, high reliability, etc. Its separation efficiency (*d*₇₅<3µm) is much higher than that of a common cyclone separator. A micro-cyclone separator predominates over both a high-speed centrifugal separator and a precision backwash filter in investment cost, maintenance, operating cost, etc. Therefore, micro-cyclone separation technology is used to purify and separate scrubbing liquid (quench water/scrubbing water), so as to recycle the purified scrubbing liquid (quench water/scrubbing water). Thirdly, in response to the requirements of economy, high efficiency and operational simplicity, a coupled separation method of micro-cyclone concentration plus gravity settling is used to concentrate the catalyst concentrated liquid. Finally, the catalyst concentrated liquid is dewatered by drying or centrifugal separation to achieve recovery of the catalyst particles in solid form. The present invention has thus been accomplished on the basis of the foregoing findings.

In a first aspect of the invention, there is provided a method for purifying and separating MTO reaction gas containing catalyst fine powder, purifying scrubbing liquid (quench water/scrubbing water) and recovering catalyst fine powder, comprising
scrubbing, purifying and cooling the MTO reaction gas containing catalyst fine powder by spray scrubbing with atomized liquid drops or dynawave scrubbing, and separating and recovering the mist entrained in the scrubbed reaction gas before it leaves the scrubbing tower (quench tower/water scrubbing tower) and the catalyst fine powder that is not scrubbed off by using a cyclone tube or a cyclone plate, so as to purify the reaction gas containing catalyst fine powder;
subjecting the scrubbing liquid (quench water/scrubbing water) containing catalyst after the scrubbing to solid-liquid separation in a sealed enclosure, so as to remove the catalyst particles in the scrubbing liquid (quench water/scrubbing water) wherein the solid content of the scrubbing liquid before separation is 30-400 mg/L, and that after separation is 10-50 mg/L and wherein the scrubbing liquid containing catalyst fine powder after separation is 1-10 vol.% of the flow of the scrubbing liquid containing catalyst fine powder;
treating the purified scrubbing liquid (quench water/scrubbing water) after the solid-liquid separation by removing methanol by subsequent heat exchange, stripping and cooling in subsequent devices and then recycling the liquid, so as to ensure the continuous and steady operation of subsequent heat exchangers and strippers;
further concentrating the concentrated liquid containing catalyst after the solid-liquid separation, so as to obtain higher concentration of catalyst slurry;
treating the further concentrated catalyst slurry by drying or centrifugal dewatering, so as to recover the catalyst fine powder in solid form;
sending the reaction gas generated by drying to a torch for burning or recovering it by condensation in a condenser; and
sending the purified scrubbing liquid (quench water/scrubbing water) generated in the process of centrifugal dewatering to the scrubbing tower (quench tower/water scrubbing tower) for repeated use.

Preferably, the particle content of the catalyst fine powder entrained in the MTO reaction gas is 1-500 mg/m³, the catalyst particle size is 0.1-30µm, and the catalyst skeletal density is 1.1-3 kg/m³.

The purification and cooling of the reaction gas is carried out by scrubbing with atomized liquid drops or dynawave scrubbing.

Preferably, the mist entrained in the scrubbed reaction gas before it leaves the scrubbing tower (quench tower/water scrubbing tower) and the catalyst fine powder that is not scrubbed off may be separated and recovered using a cyclone gas-solid/liquid separator or a cyclone plate gas-liquid separator. During separation, since the solid particles are encapsulated by liquid membrane, fine catalyst particles smaller than 3µm may also be removed effectively, and the energy consumption for separation is no more than 0.005MPa.

Preferably, no less than 99% of the catalyst particles are removed after the catalyst fine powder particles entrained in the MTO reaction gas are scrubbed and cyclone separated on top of the tower.

Preferably, all or part of the scrubbing liquid (quench water/scrubbing water) containing catalyst fine powder is first clarified, wherein the solid-liquid separation in the clarification process may be carried out by a micro-cyclone separation method.

Preferably, the solid-liquid separation in the clarification process of all or part of the scrubbing liquid (quench water/scrubbing water) containing catalyst fine powder may also be carried out by direct filtration separation using a high precision filter directly.

Preferably, the solid content of the scrubbing liquid (quench water/scrubbing water) is 30-400 mg/L before clarification, the solid content of the scrubbing liquid (quench water/scrubbing water) is 10-50 mg/L after clarification, and the energy consumption of the separation process is no more than 0.2MPa.

Preferably, all of the scrubbing liquid (quench water/scrubbing water) containing catalyst fine powder is subjected to micro-cyclone separation or precision filtration separation, and the purified scrubbing liquid (quench water/scrubbing water) is sent to subsequent devices for heat exchange, stripping, etc. before returned for recycling.

Preferably, part of the scrubbing liquid (quench water/scrubbing water) containing catalyst fine powder is subjected to micro-cyclone separation or precision filtration separation, and the purified scrubbing liquid (quench water/scrubbing water) is returned to the upper part of the bottom liquid phase in the quench tower. After an equilibrium concentration is reached, the scrubbing liquid is sent to subsequent devices for heat exchange, stripping, etc. before returned for recycling.

Preferably, the catalyst-containing solid phase concentrated liquid resulting from the clarification process is further treated in a subsequent separation and concentration device, wherein the solid phase concentrated liquid is 1-10 vol.% of the flow of the scrubbing liquid (quench water/scrubbing water) for clarifying treatment.

Preferably, the catalyst-containing solid phase concentrated liquid may be further concentrated by a coupled separation method of micro-cyclone separation and gravity settling.

Preferably, the catalyst-containing solid phase concentrated liquid may also be further concentrated by a coupled separation method of micro-cyclone separation, filtration and gravity settling.

Preferably, the further concentration of the catalyst-containing solid phase concentrated liquid results in a catalyst slurry with a solid content of 30-70 vol.%, wherein the clear liquid generated in the separation process is returned to the bottom of the scrubbing tower (quench tower/water scrubbing tower), and the energy consumption of this separation process is no more than 0.25MPa.

Preferably, the catalyst slurry of 30-70 vol.% is further dewatered so as to recover the catalyst in solid phase state.

Preferably, the dewatering process may be carried out by spray granulation drying or steam rotary tube drying, and the water content of the catalyst particles after the drying treatment is no more than 5%.

Preferably, the gas generated in the drying and dewatering process may be sent to a torch directly for burning.

Preferably, the gas generated in the drying and dewatering process may be alternatively condensed by a condenser and then returned to the scrubbing tower (quench tower/water scrubbing tower) for reuse.

Preferably, the dewatering process may be alternatively carried out by centrifugal dewatering, and the water content of the catalyst particles after dewatering is no more than 10 vol.%.

Preferably, the purified scrubbing liquid (quench water/scrubbing water) resulting from the centrifugal dewatering is returned to the scrubbing tower (quench tower/water scrubbing tower) for recycling.

Preferably, after a combinatorial separation process of micro-cyclone separation, precision filtration separation, gravity settling, drying dewatering or centrifugal dewatering, etc., more than 96% of the catalyst fine powder in the scrubbing liquid (quench water/scrubbing water) is recovered, and the energy consumption for operating the system is no more than 0.6MPa.

Specifically, the method for purifying and separating MTO reaction gas containing catalyst fine powder, purifying scrubbing liquid (quench water/scrubbing water), and dewatering and recovering catalyst fine powder according to the invention comprises the following steps.

First, the MTO reaction gas containing catalyst fine powder is scrubbed with atomized liquid drops or dynawave, purified and cooled, wherein the content of the catalyst particles entrained in the reaction gas is 1-500mg/m³, the catalyst particle size is 0.1-10µm, and the catalyst skeletal density is 2.1-2.5 kg/m³. Then, the mist entrained in the scrubbed reaction gas before it leaves the scrubbing tower (quench tower/water scrubbing tower) and the catalyst fine powder that is not scrubbed off are separated and recovered. The above processes remove no less than 99% of the catalyst fine powder particles entrained in the reaction gas.

Secondly, the catalyst that enters the scrubbing liquid (quench water/scrubbing water) is separated to prevent the catalyst fine powder entrained in the scrubbing liquid (quench water/scrubbing water) from entering and clogging subsequent devices for heat exchange, stripping, etc., and thus interfering the long cycle operation of the whole system. In this process, a micro-cyclone separation method is used to purify and separate 35% of the circulating scrubbing liquid (quench water/scrubbing water) (wherein the solid content of the circulating scrubbing liquid (quench water/scrubbing water) is 200-300 mg/L, that of the clarified and purified scrubbing liquid (quench water/scrubbing water) is 20-30 mg/L, and the energy consumption of the separation process is no more than 0.2MPa). The clarified and purified liquid is returned to the upper part of the liquid phase space in the scrubbing tower (quench tower/water scrubbing tower), and then pumped to subsequent devices for heat exchange and stripping before it is returned for recycling (wherein the equilibrium concentration of the scrubbing liquid (quench water/scrubbing water) in the whole circulation system is about 210-230mg/L).

Finally, the catalyst that enters the scrubbing liquid (quench water/scrubbing water) is separated and recovered, so that the environmental problem resulting from diffusion of fine solid particles is avoided, and recovery of relevant resource is achieved. In this process, the catalyst concentrated liquid generated in the micro-cyclone clarification is first further concentrated to a solid content of 30-70% by a coupled separation method of micro-cyclone concentration and gravity settling, and finally the resulting catalyst concentrated slurry is dewatered in a centrifuge. After dewatering, the catalyst particles (with a water content of no more than 10%) are recovered in solid form, and the clear scrubbing liquid (quench water/scrubbing water) generated during dewatering is returned to the scrubbing tower (quench tower/water scrubbing tower) for reuse.

Preferably, after the MTO reaction gas containing catalyst fine powder is treated by the wet scrubbing plus the cyclone liquid removal and then the micro-cyclone separation, no less than 99% of the catalyst fine powder particles entrained in the reaction gas are removed, and the energy consumption of the cyclone separation is no more than 0.005MPa.

Preferably, no less than 90% of the catalyst fine powder is removed in the micro-cyclone separation process for purifying the scrubbing liquid (quench water/scrubbing water), and the separation precision is 1-2µm.

Preferably, after the micro-cyclone separation of the catalyst-containing scrubbing liquid (quench water/scrubbing water) that is 35% of the circulating amount of the scrubbing liquid (quench water/scrubbing water), the solid content of the final circulating scrubbing liquid (quench water/scrubbing water) is no more than 250mg/L, and no less than 98% of the scrubbing liquid (quench water/scrubbing water) is recycled.

Reference will now be made to the drawings.

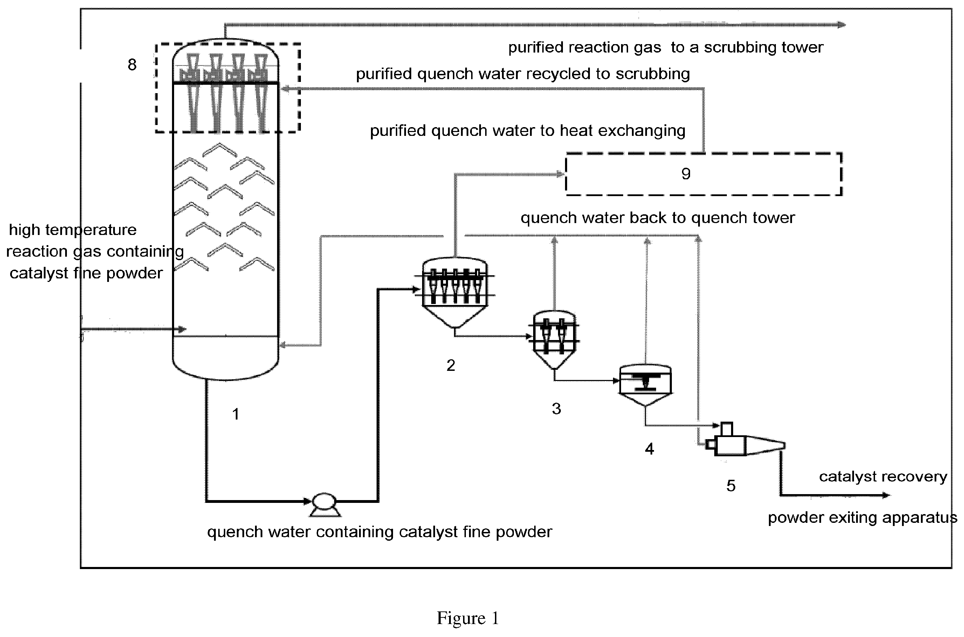

Fig. 1 is a schematic flow chart of combined purification and separation of MTO reaction gas containing catalyst fine powder according to an embodiment of the invention. This flow chart shows the micro-cyclone separation and purification of all of the scrubbing liquid (quench water/scrubbing water). As shown in Fig. 1, a high-temperature MTO reaction gas containing catalyst fine powder is fed from the bottom of a scrubbing tower (quench tower/water scrubbing tower) 1; a plurality of tiers of spray droplets arranged in the scrubbing tower (quench tower/water scrubbing tower) downwardly from the tower top come into contact in counter direction with the reaction gas moving upwardly; in this section, the process for cooling the reaction gas and removing the catalyst particles from the reaction gas by scrubbing is completed; subsequently, the mist entrained in the reaction gas before it leaves the scrubbing tower (quench tower/water scrubbing tower) and the catalyst fine powder that is not scrubbed off are separated and recovered by cyclone gas-solid/liquid separators 8 installed on the top of the tower; the above two processes fulfill purification of the reaction gas, and the catalyst fine powder enters the scrubbing liquid (quench water/scrubbing water); all of the circulating scrubbing liquid (quench water/scrubbing water) is pumped by a circulating pump into a micro-cyclone clarifier 2 for micro-cyclone separation; the purified and separated scrubbing liquid (quench water/scrubbing water) is sent to subsequent heat exchange and stripping devices 9 for treatment, and then returned to the scrubbing tower (quench tower/water scrubbing tower) for recycling; the catalyst-containing concentrated liquid that is separated out in the micro-cyclone purification and separation process enters a micro-cyclone concentrator 3 and a settling concentrating tank 4 for further concentration; the clear liquid generated in this process is returned to the scrubbing tower (quench tower/water scrubbing tower) for reuse; the further concentrated catalyst slurry is sent to a centrifugal dehydrator 5 for dewatering; after dewatering, the catalyst fine powder is recovered in solid form, and the clear liquid generated in the dewatering process is returned to the quench tower for reuse; and such treatment is repeated in cycle as described above.

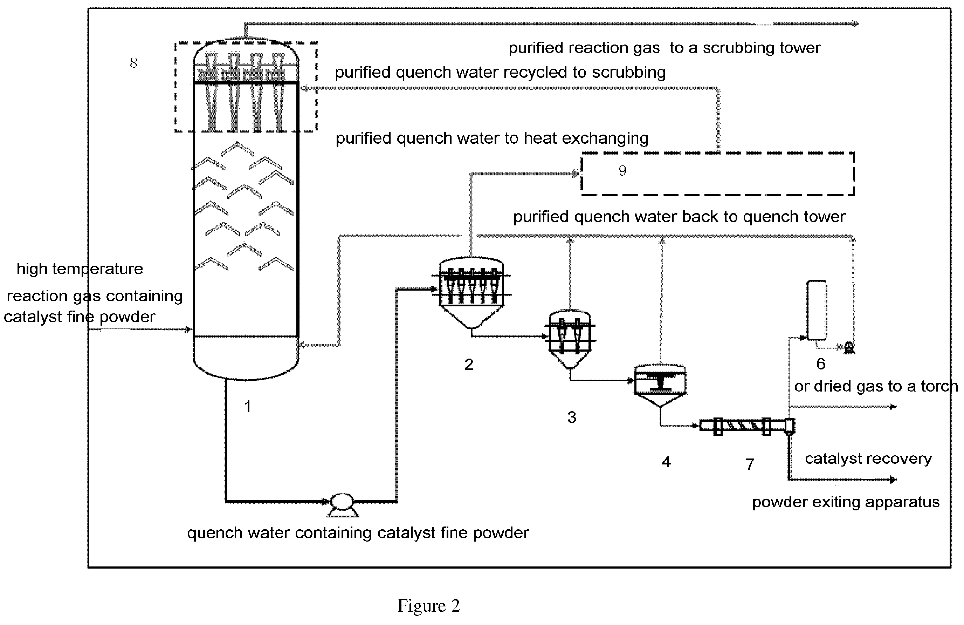

Fig. 2 is a schematic flow chart of combined purification and separation of MTO reaction gas containing catalyst fine powder according to another embodiment of the invention. As shown in Fig. 2, the difference between this flow and that shown in Fig. 1 is that the centrifugal dehydrator 5 is replaced by a spray granulation dryer 7; the catalyst slurry resulting from further concentration by the micro-cyclone concentrator 3 and the settling concentrating tank 4 is sent to the spray granulation dryer for drying; the dried catalyst fine powder is recovered, and the waste gas generated in the drying process is sent to a condenser 6 for condensation and then returned to the quench tower for reuse; alternatively, the waste gas is sent to a torch directly for burning.

According to this flow, only a portion of the scrubbing liquid (quench water/scrubbing water) needs to be subjected to solid-liquid micro-cyclone separation, and a part of the quench water is drawn from the bottom of the scrubbing tower (quench tower/water scrubbing tower) to be put into circulation directly.

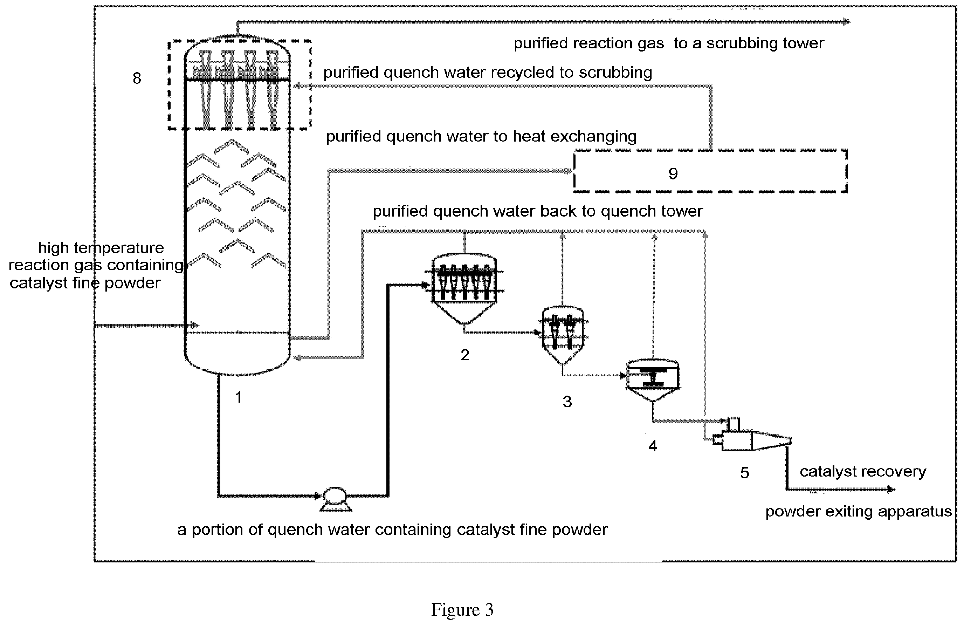

Fig. 3 is a schematic flow chart of combined purification and separation of MTO reaction gas containing catalyst fine powder according to yet another embodiment of the invention. As shown in Fig. 3, the difference between this flow and that shown in Fig. 1 is that a portion (about 35%) of the circulating scrubbing liquid (quench water/scrubbing water) is pumped into the micro-cyclone clarifier 2 for treatment, and the clarified scrubbing liquid (quench water/scrubbing water) is first returned to the upper part of the liquid phase space in the quench tower, and then sent to subsequent devices for heat exchange and stripping before returned to the scrubbing tower (quench tower/water scrubbing tower) for recycling. This flow features a constantly low equilibrium concentration of the catalyst fine powder in the scrubbing liquid (quench water/scrubbing water) to ensure the continuous operating cycle of the subsequent devices and a small processing throughput of the circulating scrubbing liquid (quench water/scrubbing water) to save energy and reduce resource consumption.

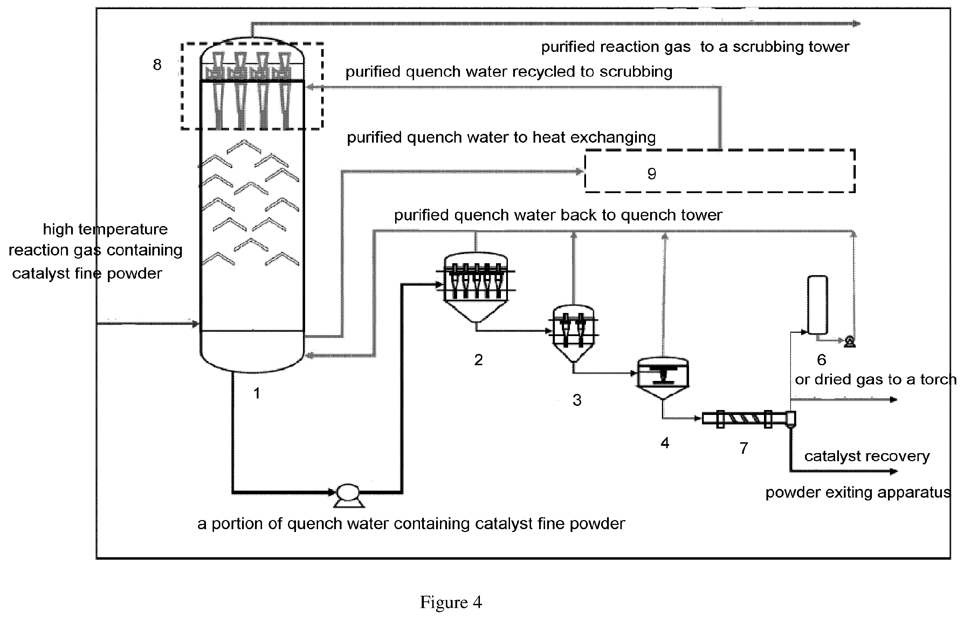

Fig. 4 is a schematic flow chart of combined purification and separation of MTO reaction gas containing catalyst fine powder according to yet another embodiment of the invention. As shown in Fig. 4, the difference between this flow and that shown in Fig. 2 is that a portion (about 35%) of the circulating scrubbing liquid (quench water/scrubbing water) is pumped into the micro-cyclone clarifier 2 for treatment, and the clarified scrubbing liquid (quench water/scrubbing water) is first returned to the upper part of the liquid phase space in the quench tower, and then sent to subsequent devices for heat exchange and stripping before returned to the scrubbing tower (quench tower/water scrubbing tower) for recycling, which is the same as the method in flow of Fig. 3, while the subsequent concentration and dewatering processes are carried out using the method in flow of Fig. 2.

Now that a part of the solid particles are discharged from the apparatus all along with the discharge of a part of the solution in the scrubbing tower (quench tower/water scrubbing tower) for cyclone separation, the solid content of the circulating scrubbing liquid (quench water/scrubbing water) in the whole apparatus is kept at an equilibrium level to the extent that the requirement of circulating use is satisfied. This leads to reduced processing throughput of the micro-cyclone separator for the scrubbing liquid (quench water/scrubbing water) and lowered energy consumption for separation.

Since quench water is generally water that is atomized and sprayed for scrubbing and cooling, micro-cyclone separation of a part of the quench liquid may not only ensure that the solid content of the circulating scrubbing liquid (quench water/scrubbing water) is kept at a suitable equilibrium level, but also reduce the energy consumption for separation and the occupation area. Therefore, Fig. 3 shows the most preferred flow for combined purification and separation of MTO reaction gas containing catalyst fine powder.

The main advantages of the process of the invention include:
According to the invention, a coupled method of counter-current scrubbing and cyclone liquid removal is used to cool and purify MTO reaction gas containing catalyst fine powder, wherein the method takes advantage of the feature that the fine catalyst particles are encapsulated by the atomized liquid drops, so that the removal rate of the catalyst particles is promoted effectively. Secondly, a micro-cyclone separation method is used to separate and purify the circulating scrubbing liquid (quench water/scrubbing water), so that the efficiency of circulating use of the scrubbing liquid (quench water/scrubbing water) is enhanced, and the use cycle of the apparatus is prolonged. Finally, the concept of coupling stages of concentration and dewatering steps is used for effective recovery of the catalyst fine powder, leading to recovery of the resource, reduced investment and maintenance cost, as well as lowered energy consumption. According to the invention, a coupled process comprised of scrubbing/cooling and cyclone gas-solid (liquid) separation is used for treating MTO reaction gas containing catalyst fine powder, and a coupled process comprised of once clarification, twice concentration and final drying or centrifugal dewatering for recovery of the catalyst is used for treating scrubbing liquid (quench water/scrubbing water), so that the catalyst entrained in the MTO reaction gas can be removed effectively. In addition, the treatment processes are carried out in a sealed enclosure. Thus, the risk of methanol in the scrubbing liquid (quench water/scrubbing water) is eliminated, the continuous circulation of the scrubbing liquid (quench water/scrubbing water) is ensured, the catalyst fine powder is recovered efficiently, and the continuous and steady operation of subsequent devices for heat exchange and stripping is guaranteed. Owing to simple operation, low energy consumption and high recovery of resources, the invention is suitable for use in purification and separation processes for treating MTO reaction gas as well as other fume and reaction gas entraining fine solid particles.

## Claims

1. A process for optimized combination of purification and separation of MTO reaction gas containing catalyst fine powder, comprising
(a) scrubbing, purifying and cooling the MTO reaction gas containing catalyst fine powder by spray scrubbing with atomized liquid drops or dynawave scrubbing, and separating and recovering the mist entrained in the scrubbed reaction gas before it leaves the water scrubbing tower and the catalyst fine powder that is not scrubbed off by using a cyclone tube or a cyclone plate, so as to purify the reaction gas containing catalyst fine powder;
(b) subjecting the scrubbing water containing catalyst after the scrubbing to solid-liquid separation in a sealed enclosure by micro-cyclone separation or filtration separation, so as to remove the catalyst particles in the scrubbing water, wherein the solid content of the scrubbing liquid before separation is 30-400 mg/L, and after separation is 10-50 mg/L and wherein the scrubbing liquid containing catalyst fine powder after separation is 1-10 vol.% of the flow of the scrubbing liquid containing catalyst fine powder;
(c) treating the purified scrubbing water after the solid-liquid separation by removing methanol by subsequent heat exchange, stripping and cooling in subsequent devices and then recycling the liquid, so as to ensure the continuous and steady operation of subsequent heat exchangers and strippers;
(d) further concentrating the concentrated liquid containing catalyst after the solid-liquid separation, so as to obtain higher concentration of catalyst slurry;
(e) treating the further concentrated catalyst slurry by drying or centrifugal dewatering, so as to recover the catalyst fine powder in solid form;
(f) sending the reaction gas generated by drying to a torch for burning or recovering it by condensation in a condenser; and
(g) sending the purified scrubbing water generated in the process of centrifugal dewatering to the water scrubbing tower for repeated use.

2. The process of Claim 1, wherein the catalyst fine powder particles entrained in the MTO reaction gas containing catalyst fine powder have a content of 1-500mg/m³ and a particle size of 0.1-30µm, and the catalyst skeletal density is 1.1~3 kg/m³.

3. The process of Claim 1, wherein the scrubbing liquid containing catalyst fine powder after separation is further concentrated to a solid content of 30-70 vol.% by coupled separation of micro-cyclone separation with gravity settling, or micro-cyclone separation with filtration and gravity settling.

4. The process of Claim 3, wherein the concentrated scrubbing liquid containing catalyst fine powder after separation with a solid content of 30-70 vol.% is dried or dewatered in a centrifuge, wherein the drying is spray granulation drying or rotary steam tube drying, the dried catalyst is recovered, and the dried gas is sent to a torch or recovered after condensed.

5. The process of Claim 4, wherein after the concentrated scrubbing liquid containing catalyst fine powder after separation with a solid content of 30-70 vol.% is dried, the water content of the catalyst particles is no more than 5 vol.%; alternatively, after the concentrated scrubbing liquid containing catalyst fine powder after separation with a solid content of 30-70 vol.% is dewatered in a centrifuge, the water content of the catalyst particles is no more than 10 vol.%.

## Patentansprüche

1. Verfahren zur optimierten Kombination von Aufreinigung und Trennung von MTO-Reaktionsgas enthaltenden Katalysator-Feinpulver, umfassend
(a) Auswaschen, Aufreinigen und Kühlen des MTO-Reaktionsgases enthaltend Katalysator-Feinpulver durch Sprühwaschen mit zerstäubten Flüssigkeitstropfen oder Dynawave-Waschen, und Trennen und Wiedergewinnen des in dem gereinigten Reaktionsgas mitgeführten Nebels, bevor es den Wasser-Waschturm verlässt, und des Katalysator-Feinpulvers, das nicht ausgewaschen ist, durch Verwendung einer Zyklonröhre oder einer Zyklonplatte, um das Katalysator- Feinpulver, welches das Reaktionsgas enthält, aufzureinigen;
(b) Unterziehen des Waschwasser enthaltenden Katalysators nach dem Waschen einer Feststoff-Flüssigkeit-Trennung in einem abgedichteten Behälter mittels Mikro-Zyklontrennung oder Filtrationstrennung, um die Katalysatorteilchen aus dem Waschwasser zu entfernen, wobei der Feststoffgehalt der Waschflüssigkeit vor Trennung 30-400 mg/l beträgt und nach Trennung 10-50 mg/l beträgt und wobei das Waschflüssigkeit enthaltende Katalysator-Feinpulver nach Trennung 1-10 Vol.% des Stroms des Waschflüssigkeit enthaltenden Katalysator-Feinpulvers beträgt;
(c) Behandeln des aufgereinigten Waschwassers nach der Feststoff-Flüssigkeit-Trennung durch Entfernen von Methanol durch anschließenden Wärmetausch, Strippen und Kühlen in nachfolgenden Vorrichtungen und dann Zurückführen der Flüssigkeit, um den kontinuierlichen und gleichmäßigen Betrieb nachfolgender Wärmetauscher und Abstreifer sicherzustellen;
(d) weiteres Konzentrieren des konzentrierte Flüssigkeit enthaltenden Katalysators nach der Feststoff-Flüssigkeit-Trennung, um so eine höhere Konzentration von Katalysatorschlamm zu erhalten;
(e) Behandeln des weiter konzentrierten Katalysatorschlamms durch Trocknen oder zentrifugales Entwässern, um so das Katalysator-Feinpulver in fester Form wiederzugewinnen;
(f) Senden des durch Trocknen gewonnenen Reaktionsgases an eine Fackel zum Brennen oder Wiedergewinnen des Gases durch Kondensieren in einem Kondensator; und
(g) Senden des aufgereinigten Waschwassers, erzeugt in dem Verfahren der zentrifugalen Entwässerung, an den Wasser-Waschturm zur wiederholten Verwendung.

2. Verfahren nach Anspruch 1, wobei die Katalysator-Feinpulverteilchen, mitgeführt in dem Katalysator-Feinpulver enthaltenden MTO-Reaktionsgas, einen Gehalt von 1-500 mg/m³ und eine Teilchengröße von 0,1-30 µm haben und die Katalysator-Skelettdichte 1,1~3 kg/m³ beträgt.

3. Verfahren nach Anspruch 1, wobei das Waschflüssigkeit enthaltende Katalysator-Feinpulver nach Trennen durch gekoppelte Trennung von Mikrozyklon-Trennung mit Schwerkraftabsetzung oder Mikrozyklon-Trennung mit Filtration und Schwerkraftabsetzung weiter zu einem Feststoffgehalt von 30-70 Vol.% konzentriert wird.

4. Verfahren nach Anspruch 3, wobei das konzentrierte Waschflüssigkeit enthaltende Katalysator-Feinpulver nach dem Trennen mit einem Feststoffgehalt von 30-70 Vol.% in einer Zentrifuge getrocknet oder entwässert wird, wobei die Trocknung Sprühgranulationstrocknung oder Rotationsdampfrohrtrocknung ist, der getrocknete Katalysator zurückgewonnen wird und das getrocknete Gas an eine Fackel gesendet oder nach dem Kondensieren zurückgewonnen wird.

5. Verfahren nach Anspruch 4, wobei, nachdem das konzentrierte Waschflüssigkeit enthaltende Katalysator-Feinpulver nach dem Trennen mit einem Feststoffgehalt von 30-70 Vol.% getrocknet ist, der Wassergehalt der Katalysatorteilchen nicht mehr als 5 Vol.% beträgt; alternativ, nachdem das konzentrierte Waschflüssigkeit enthaltende Katalysator-Feinpulver nach dem Trennen mit einem Feststoffgehalt von 30-70 Vol.% in einer Zentrifuge entwässert ist, der Wassergehalt der Katalysatorteilchen nicht mehr als 10 Vol.% beträgt.

## Revendications

1. Procédé permettant une combinaison optimisée de purification et de séparation d'un gaz de réaction MTO contenant une fine poudre catalytique, comprenant
- (a) le lavage, la purification et le refroidissement du gaz de réaction MTO contenant de la fine poudre catalytique par lavage par pulvérisation à l'aide de gouttes de liquide atomisées ou d'un épurateur-laveur Dynawave, et la séparation et la récupération de la brume entrainée dans le gaz de réaction lavé avant qu'elle ne quitte la tour de lavage humide et la poudre fine catalytique qui n'est pas lavée à l'aide d'un tube cyclonique ou d'une plaque cyclonique, de sorte à purifier le gaz de réaction contenant la fine poudre catalytique ;
- (b) le fait de soumettre l'eau de lavage contenant le catalyseur après le lavage à la séparation solide-liquide dans une enceinte fermée hermétiquement par séparation micro-cyclonique ou séparation par filtration, de sorte à éliminer les particules catalytiques dans l'eau de lavage,
dans lequel la teneur en solides du liquide de lavage avant séparation est de 30-400 mg/L, et après séparation est de 10-50 mg/L et dans lequel le liquide de lavage contenant la poudre fine catalytique après séparation est de 1-10 % en volume du flux du liquide de lavage contenant la fine poudre catalytique ;
- (c) le traitement de l'eau de lavage purifiée après la séparation solide-liquide par l'élimination du méthanol par échange thermique, épuisement et refroidissement ultérieurs dans les dispositifs suivants et ensuite le recyclage du liquide, de sorte à garantir le fonctionnement continu et régulier des échangeurs de chaleurs et des épuiseurs consécutifs ;
- (d) la concentration ensuite du liquide concentré contenant le catalyseur après la séparation solide-liquide, de sorte à obtenir une concentration plus forte des boues catalytiques ;
- (e) le traitement des boues catalytiques davantage concentrées par séchage ou déshydratation centrifuge, de sorte à récupérer la poudre fine catalytique sous forme solide ;
- (f) l'envoi du gaz de réaction généré en le séchant avec une torche pour le brûler ou le récupérer par condensation dans un condensateur ; et
- (g) l'envoi de l'eau de lavage purifiée générée dans le procédé de déshydratation centrifuge vers la tour de lavage humide pour une utilisation répétée.

2. Procédé selon la revendication 1, dans lequel les particules de fine poudre catalytique entraînées dans le gaz de réaction MTO contenant la fine poudre catalytique ont une teneur de 1 à 500 mg/m³ et une taille des particules de 0,1 à 30 µm, et la densité squelettique est de 1,1∼3 kg/m³.

3. Procédé selon la revendication 1, dans lequel le liquide de lavage contenant la fine poudre catalytique après séparation est en outre concentré à une teneur en solides de 30 à 70 % en volume par séparation couplée d'une séparation micro-cyclonique avec cristallisation par densité ou séparation micro-cyclonique avec filtration et cristallisation par densité.

4. Procédé selon la revendication 3, dans lequel le liquide de lavage concentré contenant la fine poudre catalytique après séparation avec une teneur en solides de 30 à 70 % en volume est séché ou déshydraté dans une centrifugeuse, dans lequel le séchage est un séchage par granulation par pulvérisation ou séchage par tube à vapeur rotatif, le catalyseur séché est récupéré et le gaz séché est envoyé vers une torche ou récupéré après avoir été condensé.

5. Procédé selon la revendication 4, dans lequel après que le liquide de lavage concentré contenant la poudre fine catalytique après séparation avec une teneur en solides de 30 à 70 % en volume est séché, la teneur en eau des particules catalytiques ne dépasse pas 5 % en volume ; en variante, après que le liquide de lavage concentré contenant la poudre fine catalytique après séparation avec une teneur en solides de 30 à 70 % en volume est déshydraté dans une centrifugeuse, la teneur en eau des particules catalytiques ne dépasse pas 10 % en volume.
